# EUROPEAN PATENT SPECIFICATION

(11) **EP 1 656 899 B1**
(45) Date of publication and mention of the grant of the patent: **17.09.2008**
(21) Application number: 05256460.6
(22) Date of filing: 18.10.2005
(51) Int. Cl.: A61B 17/74, A61B 17/86

(54) **Glenoid instrumentation anti-migration threaded fastener**
Antimigrationsbefestigungselement mit Gewinde
Fixation filetée anto-migration pour une instrumentation glénoidienne

(30) Priority: 28.06.2005 US 168039; 28.06.2005 US 168709; 28.06.2005 US 168737; 12.11.2004 US 627266 P
(43) Date of publication of application: 17.05.2006
(73) Proprietor: DePuy Products, Inc., Warsaw, IN 46581 (US)
(72) Inventor: Reber, Erik W, Warsaw, IN 46582 (US); Vanderlinden, Timothy P, Warsaw, IN 46582 (US); Fisher, James M, West Chester, OH 45069 (US)
(74) Representative: Belcher, Simon James

(56) References cited:
- EP-A- 1 356 777
- US-A- 4 733 654
- US-A- 5 116 336
- US-A- 6 022 352
- US-A1- 2004 230 195
- US-B1- 6 319 254
- PATENT ABSTRACTS OF JAPAN vol. 2002, no. 09, 4 September 2002 (2002-09-04) & JP 2002 153479 A (TAKENAKA:KK), 28 May 2002 (2002-05-28)

## Description

The present invention relates generally to the field of orthopaedics, and more particularly, to a device for use in treating orthopaedic trauma.

The skeletal system includes many long bones that extend from the human torso. These long bones include the femur, fibula, tibia, humerus, radius and ulna. These long bones are particularly exposed to trauma from accidents, and as such often are fractured during such trauma and may be subject to complex devastating fractures.

Automobile accidents, for instance, are a common cause of trauma to long bones. In particular, the femur and tibia frequently fracture when the area around the knee is subjected to a frontal automobile accident.

Often the distal end or proximal portions of the long bone, for example the femur and the tibia, are fractured into several components and must be realigned. Mechanical devices, commonly in the forms of pins, plates, screws, nails, wires and external devices are commonly used to attach fractured long bones. The pins, plates, wires, nails and screws are typically made of a durable material compatible to the human body, for example titanium, stainless steel or cobalt chromium.

Fractures of the long bone are typically secured into position by at least one of three possible techniques.

The first method is the use of intramedullary nails that are positioned in the intramedullary canal of those portions of the fractured bone.

A second method of repairing fractured bones is the use of internal bone plates that are positioned under the soft tissue and on the exterior of the bone and bridges the fractured portion of the bone.

Another method of securing fractured bones in position is the use of external fixators. These external fixators have at least two general categories. In one category the fixator is generally linear with a first portion of the fixator to connect to a first fracture segment of the bone and a second fracture segment of the fixator to connect to the second fracture segment of the bone. A first series of bone screws or pins are first connected to the fixator and then into the first portion of the bone. Then a second series of screws or pins are connected to the fixator and then to the second fracture segment of the bone, thereby securing the first portion fracture segment of the bone to the second portion of the bone.

A second method of external fixation is through the use of a ring type fixator that uses a series of spaced-apart rings to secure the bone. For example, an upper ring and a lower ring are spaced apart by rods. A plurality of wires is placed through the long bone and is connected on each end of the long bone by the ring. The wires are then tensioned much as a spoke in a bicycle are tightened, thereby providing for a rigid structure to support the first fracture segment portion of the bone. Similarly, a plurality of wires are positioned through the second fracture segment of the bone and are secured to and tensioned by the lower ring to provide a rigid fixation of the second fracture segment of the bone bridging the fracture site.

There are a variety of devices used to treat femoral fractures. Fractures of the neck, head or intertrochanter of the femur have been successfully treated with a variety of compression screw assemblies which include generally a compression plate having a barrel member, a lag screw and a compressing screw. The compression plate is secured to the exterior of the femur and the barrel member is inserted into a predrilled hole in the direction of the femoral head.

The lag screw, which has a threaded end and a smooth portion is inserted through the barrel member so that it extends across the break and into the femoral head. The threaded portion engages the femoral head. The compressing screw connects the lag screw to the plate. By adjusting the tension of the compressing screw the compression (reduction) of the fracture can be adjusted. The smooth portion of the lag screw must be free to slide through the barrel member to permit the adjustment of the compression screw.

Subtrochanteric and femoral shaft fractures have been treated with the help of intramedullary rods which are inserted into the marrow canal of the femur to immobilize the femur parts involved in fractures. A single angled cross-nail or locking screw is inserted through the femur and the proximal end of the intramedullary rod. In some varieties, one or two screws may also be inserted through the femoral shaft and through the distal end of the intramedullary rod. The standard intramedullary rods have been successfully employed in treating fractures in lower portions of the femoral shaft.

Trochanteric nails for use in preparing femoral neck fractures utilize a screw in the form of, for example, a lag screw. The lag screws have several different problems in use that are generally related to the lag screw not remaining in the proper position with respect to intramedullary nail during the operating live of an implant. For example, the lag screw may cut proximally through the femoral neck and head causing the neck and head to move out its operating position in cooperation with the acetabulum. Such a movement be render the patient non-ambulatory. Another issue that may occur with lag screws is medial migration of a lag screw through the femoral head and into the pelvic cavity. Yet another issue with an intramedullary nail lag screw is lateral migration or lateral pullout of the screw from the long bone.

Yet another problem with lag screws in trochanteric nail applications is the problem of neck collapse. Early after the implantation of the trochanteric nail, for example, at the first weight-bearing instance of the patient, the head of the femur may move distally due to a phenomenon known as neck collapse. If the lag screw does not capture enough cancellous bone in the femoral neck, the neck and head may move laterally causing the phenomenon known as neck collapse and creating a leg length and other issues for the patient.

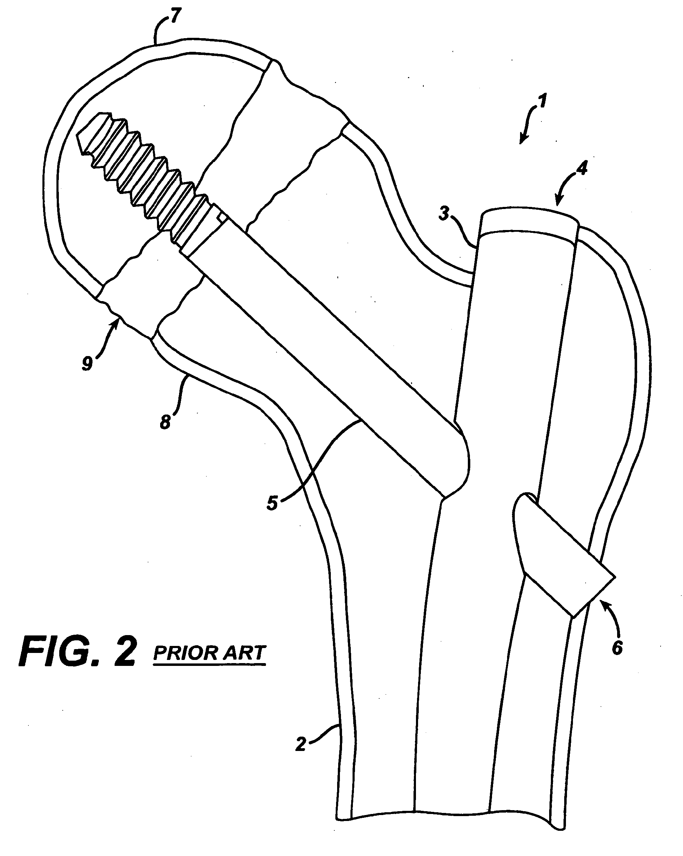

Referring now to FIG. 2, a prior art intramedullary nail assembly 1 is shown in position on femur 2. The intramedullary nail assembly 1 includes an intramedullary nail 3 that is positioned in the medullary canal 4 of the femur 2. A lag screw 5 is positioned through transverse opening 6 of the nail 3. The lag screw 5 is utilized to connect the head 7 and neck 8 to the remainder of the femur 2. As can be seen in FIG. 2, fracture site 9 is full of weak osteoporotic bone.

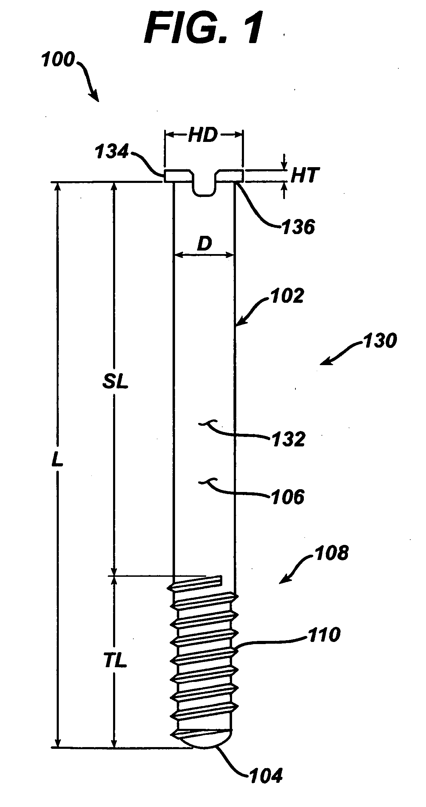

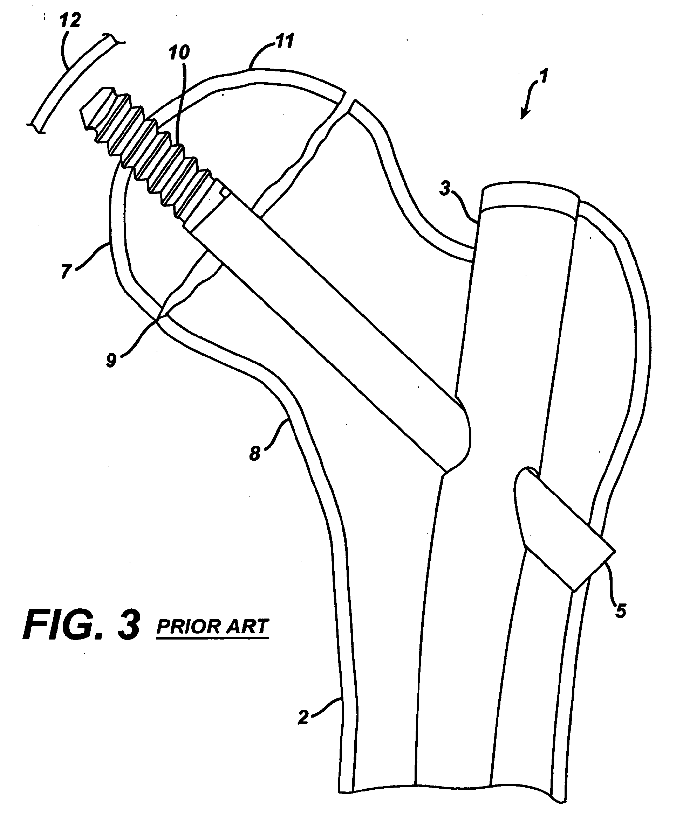

Referring now to FIG. 3, the intramedullary nail assembly 1 of FIG. 1 is shown in the femur 2 having experienced a collapse of the femoral neck 8. The threads 10 of lag screw 5 are insufficient to prevent head 7 of the femur 2 to collapse with the neck 8 causing the head 7 to migrate laterally on the patient. The screw 5 may extend as shown in FIG. 3 pass the periphery 11 of the head 7 and impinge into the acetabulum 12 causing a major problem for the patient.

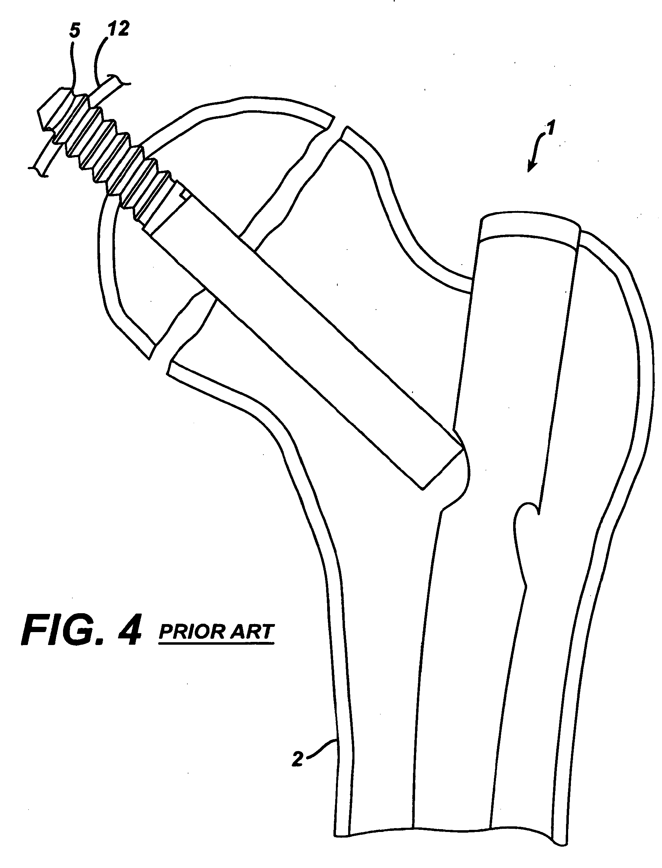

Referring now to FIG. 4, the prior art intramedullary nail assembly 1 is shown in position on a femur 2 showing medial migration of the lag screw 5 into a position where the lag screw has passed through the acetabulum 12 causing a serious problem for the patient.

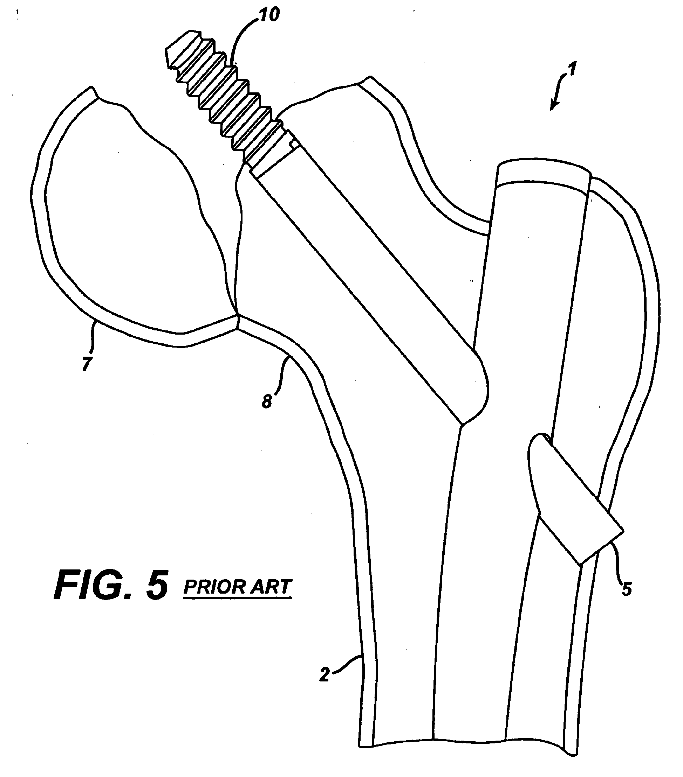

Referring now to FIG. 5, intramedullary nail assembly 1 is shown with threads 10 of the lag screw 5 having provided for cut-out of the threads 10 through the neck 8 and the head 7 of the femur 2 causing the head 7 to move distally with respect to the patient. The cut-out phenomenon, as shown in FIG. 5, causes a problem for the patient and causes the patient to no longer be ambulatory.

Attempts have been made to overcome the problem with medial migration. For example, Synthes offers a locking mechanism to prevent medial migration. To address the length for cutting proximally through the femoral neck and head on Synthes does not utilize threads and utilizes a fluted tip that is hammered into the body instead of threaded to the nail.

To overcome problems with rotation of the lag screw in the intramedullary nail Smith, Nephew and Richards on utilizes a flat in the lag screw shaft in order to use a keyed centered sleeve mechanism to prevent rotation. To prevent migration, Smith and Nephew are utilizing a locking compression screw in the distal end of the lag screw. The keyed sleeve and compression screw increase operating room time.

A trochanteric nail is available from Stryker Corporation under the trade mark Gamma which utilizes a set screw threaded down the cannulation in the nail and grooves in the shafts of the lag screw to address the issue of medial migration.

US-5116336 discloses an osteosynthetic anchor bolt for fixing a bone plate to a bone. The bolt has a shaft which is threaded at its distal end. The thread has first and second flanks with a crest between them. The angles between the crest and the first and second flanks are different from one another.

The new thread design of the present invention addresses the problems of thread cut-out and also the issue of medial migration.

According to the present invention, an orthopaedic screw is provided with a single thread form. The thread form runs along the central longitudinal axis of the screw. Starting from the proximal end, the thread starts as a flat section horizontal to the central longitudinal axis. The flat turns into an angle section rising away from the central longitudinal axis toward the distal end of the screw at an angle between 1 and 98°. The angular surface changes into a flat surface rising up from the central longitudinal axis. This flat surface is perpendicular to the central longitudinal.

The perpendicular flat surface then changes into another flat surface that is parallel to the central longitudinal axis continuing to move distally along the central longitudinal axis. The current flat surface changes into another flat surface which is perpendicular to the central longitudinal axis. This finally flat surface changes into another flat surface that is parallel to the central longitudinal axis and is co-linear to the first flat surface. This pattern is repeated over again given length diameter of the rod.

According to one embodiment of the present invention, there is provided an orthopaedic screw. The orthopaedic screw includes a shank defining an end and a periphery of the shank. A portion of the periphery defines a thread form. The thread form includes a first flank, a crest adjacent the first flank and a second flank spaced from the first flank and adjacent the crest. The crest and the first flank form a first angle between the crest and the first flank. The crest and the second flank form a second angle between the crest and the second flank. The first angle and the second angle are different from each other.

According to another embodiment of the present invention there is provided an intramedullary nail assembly for use in a medullary canal of a long bone. The assembly includes a nail for positioning at least partially in the medullary canal. The nail includes an aperture through the nail. The assembly also includes a screw fittably positioned in the aperture of the nail. The screw has a shank defining an end and a periphery of the shank. A portion of the periphery defines a thread form. The thread form includes a first flank, a crest adjacent the first flank and a second flank spaced from the first flank and adjacent the crest. The crest and the first flank form a first angle between the crest and the first flank. The crest and the second flank form a second angle between the crest and the second flank. The first angle and the second angle are different from each other.

According to yet another embodiment of the present invention there is provided an orthopaedic screw for positioning in an aperture of an intramedullary nail. The screw has a shank defining an end and a periphery of the shank. A portion of the periphery defines a thread form. The thread form includes a first flank, a crest adjacent the first flank and a second flank spaced from the first flank and adjacent the crest. The crest and the first flank form a first spatial relationship between each other. The crest and the second flank form a second spatial relationship between each other. The first spatial relationship and the second spatial relationship are asymetrical from each other.

According to a further embodiment there is provided a method for performing trauma surgery on a long bone. The method includes the steps of providing an intramedullary nail including an aperture therethrough and positioning the nail at least partially in the medullary canal. The method further includes the step of providing a screw having a shoulder and a shank defining first and second ends and a periphery thereof, a portion of the periphery defining a thread form, the thread form including a first flank, a crest adjacent the first flank and a second flank spaced from the first flank and adjacent the crest, the crest and the first flank forming a first angle therebetween, the crest and the second flank forming a second angle therebetween, the first angle and the second angle being different from each other. The method further includes the steps of positioning the screw in the aperture of the nail and advancing the screw until the shoulder is in intimate contact with the cortical wall of the long bone.

Technical advantages of the present invention include the ability to provide a lag screw with better bone purchase for use in a trochanteric nail assembly. For example, according to one aspect of the present invention, an orthopaedic screw is provided including a thread form having a first crest and a second flank. The second flank forms a right angle with the crest and the first flank includes two portions with the first portion of the first flank and crest forming a right angle therebetween. The crest and the first and second flanks thereby form a box shaped thread. This box shaped thread provides for a better bone purchase. Thus, the present invention provides for a lag screw with better bone purchase.

The technical advantages of the present invention further include the ability to provide for an increased thread peak surface area. For example, according to another aspect of the present invention, an orthopaedic screw is provided having a thread form. The thread form includes a first flank, a crest and a second flank. The second flank and the crest form a right angle therebetween and the first flank includes a first portion and a second portion. The first portion of the first flank and the crest form a right angle therebetween. Thus, the orthopaedic screw provides for a box shaped thread form. This box shaped thread form increase the thread peak surface area. Thus, the present invention provides for increased thread peak surface area.

The technical advantages of the present invention yet include a slower migration rate of the orthopaedic screw medially. For example, according to yet another aspect of the present invention, the orthopaedic screw includes a thread form having a first flank, a crest and a second flank. The second flank and the crest form a right angle, and the first flank includes a first portion and a second portion with a first portion and the crest forming a right angle therebetween. The right angle between the first portion of the first flank and the crest reduces the ability of the orthopaedic screw of the present invention to cut or to migrate medially. Thus, the present invention provides for a slower migration rate for the orthopaedic screw.

The technical advantages of the present invention further include a lower or reduced cyclic cut-out. For example, according to yet another aspect of the present invention, an orthopaedic screw is provided with a thread form having a first flank, a crest, and a second flank. The second flank and the crest form a right angle and the first flank includes a first portion and a second portion. The first portion of the first flank and the crest form a right angle therebetween. The crest, as well as the right angled flanks, provide for a lack of a cutting edge or surface to provide for the magnitude of cut-out normally experienced in an orthopaedic lag screw. Thus, the present invention provides for reduced cyclic cut-out.

The technical advantages of the present invention further include a higher torque to initiate rotation. For example, according to yet another aspect of the present invention, an orthopaedic screw is provided including a thread form having a flank, a crest and a second flank spaced from the first flank. The second flank and the crest form a right angle and the first flank includes a first portion and a second portion. The first portion of the first flank and the crest form a right angle therebetween. The right angle between the first portion of the first flank and the crest provide for a reduced or less easily rotated cutting edge for the orthopaedic screw. Thus, the present invention provides for a higher torque to initiate the rotation of the orthopaedic screw.

The technical advantages of the present invention include a lower or reduced static cut-out utilizing the orthopaedic screw of the present invention. For example, according to yet another aspect of the present invention, an orthopaedic screw is provided including a thread form having a first flank, a crest, and a second flank. The second flank and the crest form a right angle therebetween and the first flank includes a first potion and a second portion. The first portion of the first flank and the crest form a right angle therebetween. The somewhat sizable crest of the thread form of the present invention as well as the perpendicular orientation of the first portion of the first flank with respect to the crest and the perpendicular orientation cut-out second flank with respect to the crest results provide no cutting edge and require that the cancellous bone is compressed rather than cut so that the static cut-out is greatly reduced. Thus, the present invention provides for lower or reduced static cut-out.

The technical advantages of the present invention, further include a higher pullout force required when utilizing the orthopaedic screw of the present invention. For example, according to yet another aspect of the present invention, an orthopaedic screw is provided including a thread form having a crest and a first flank and a second flank. The second flank and the crest form a right angle therebetween. The second flank is adjacent to the end of the screw when pullout of the orthopaedic screw is attempted. The second flank of the screw requires the cancellous bone to be compressed and does not permit the cancellous bone to be cut. Thus, the present invention provides for a higher pullout force when utilizing the orthopaedic screw of the present invention.

The implants provided by the present invention can be used in a method for performing trauma surgery on a long bone, comprising the steps of:
providing an intramedullary nail which has an aperture extending through it;
positioning the nail at least partially in the medullary canal;
providing a screw having a shoulder and a shank defining first and second ends and a periphery thereof, a portion of the periphery defining a thread form, the thread form including a first flank, a crest adjacent the first flank and a second flank spaced from the first flank and adjacent the crest, the crest and the first flank forming a first angle therebetween, the crest and the second flank forming a second angle therebetween, the first angle and the second angle being different from each other;
positioning the screw in the aperture of the nail; and
advancing the screw until the shoulder is in intimate contact with the cortical wall of the long bone.

Embodiments of the invention will now be described by way of example with reference to the accompanying drawings, in which:
FIG. 1 is a plan view of a screw including the anti-migration thread design in accordance to an embodiment of the present invention;
FIG. 2 is a plan view partially in cross-section of a prior art intramedullary nail in position in a femur;
FIG. 3 is a plan view partially in cross-section of the prior art intramedullary nail of FIG. 2 shown with the femur experiencing a neck collapse;
FIG. 4 is a plan view partially in cross-section of the prior art intramedullary nail of FIG. 2 shown with the nail having migrated medially;
FIG. 5 is a plan view partially in cross-section of the prior art intramedullary nail of FIG. 2 shown with the neck and head of the femur having experienced cut-out;
FIG. 6 is partial plan view of another screw according to the present invention;
FIG. 7 is cross-sectional view of FIG. 6 along the line 7-7 in the direction of the arrows;
FIG. 8 is partial plan view of the screw of FIG. 1 showing the threads in even greater detail;
FIG. 9 is cross-sectional view of FIG. 8 along the line 9-9 in the direction of the arrows;
FIG. 10 is partial cross-sectional view of the screw of FIG. 1 showing the threads in even greater detail;
FIG. 10A is partial cross-sectional view of a thread form of another anti-migration screw form;
FIG. 10B is partial cross-sectional view of a thread form of another anti-migration screw form
FIG. 10C is partial cross-sectional view of a thread form of another anti-migration screw form;
FIG. 10D is partial cross-sectional view of a thread form of another anti-migration screw form;
FIG. 10E is partial cross-sectional view of a thread form of another anti-migration screw form;
FIG. 10F is partial cross-sectional view of a thread form of another anti-migration screw form;
FIG. 10G is partial cross-sectional view of a thread form of another anti-migration screw form;
FIG. 10H is partial cross-sectional view of a thread form of another anti-migration screw form;
FIG. 10I is partial cross-sectional view of a thread form of another anti-migration screw form;
FIG. 11 is partial cross-sectional view of the screw of FIG. 1 showing the threads in even greater detail;
FIG. 12 is a partial plan view partially in cross-section of a intramedullary nail assembly including the screw of FIG. 1 in accordance with another embodiment of the present invention;
FIG. 13 is a partial plan view partially in cross-section of the intramedullary nail assembly of FIG. 12 showing partial medial migration of the screw;
FIG. 14 is a plan view of a screw including an anti-migration thread design in accordance to yet another embodiment of the present invention;
FIG. 15 is cross-sectional view of FIG. 14 along the line 15-15 in the direction of the arrows;
FIG. 16 is partial cross-sectional view of the screw of FIG. 14 showing the threads in greater detail;
FIG. 17 is a plan view partially in cross-section of a intramedullary nail assembly in position in a femur including the screw of FIG. 1 and including the screw of FIG. 14 in accordance with another embodiment of the present invention;
FIG. 18 is partial plan view partially in cross-section of the intramedullary nail assembly of FIG. 17;
FIG. 19 is another partial plan view partially in cross-section of the intramedullary nail assembly of FIG. 17 showing the screws in greater detail;
FIG. 20 is yet another partial plan view partially in cross-section of the intramedullary nail assembly of FIG. 17 showing the screws in even greater detail;
FIG. 21 is a plan view partially in cross-section of another intramedullary nail assembly in position in a femur including the screw of FIG. 1 and including the screw of FIG. 14 in accordance with yet another embodiment of the present invention;
FIG. 22 is partial plan view partially in cross-section of the intramedullary nail assembly of FIG. 20;
FIG. 23 is a partial plan view partially in cross-section of an instrument for use in implanting the intramedullary nail assembly of FIG. 21 showing the antirotation screw hole being prepared;
FIG. 24 is a partial plan view partially in cross-section of the instrument of FIG. 23 showing the antirotation screw being installed;
FIG. 25 is a partial plan view partially in cross-section of the instrument of FIG. 23 showing the distal screw hole being prepared;
FIG. 26 is a partial plan view partially in cross-section of the intramedullary nail assembly of FIG. 21 showing the cap being installed; and
FIG. 27 is a flow chart for a method of performing trauma surgery in accordance with another embodiment of the present invention;

Referring to the drawings, FIGS. 1 and 8-11 show an orthopaedic screw 100 which includes a shank 102, which defines an end 104 and a periphery 106. A portion 108 of the periphery 106 defines a thread form 110.

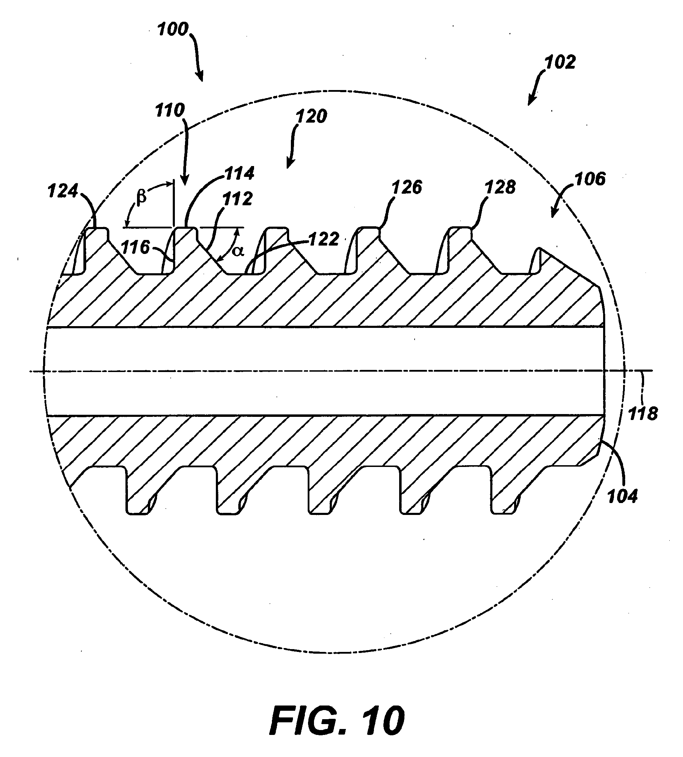

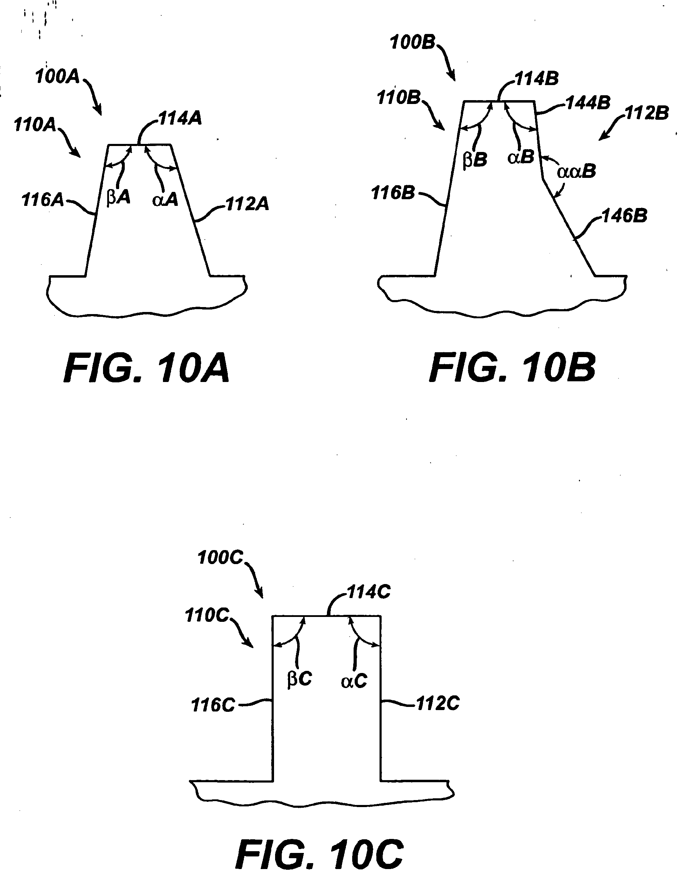

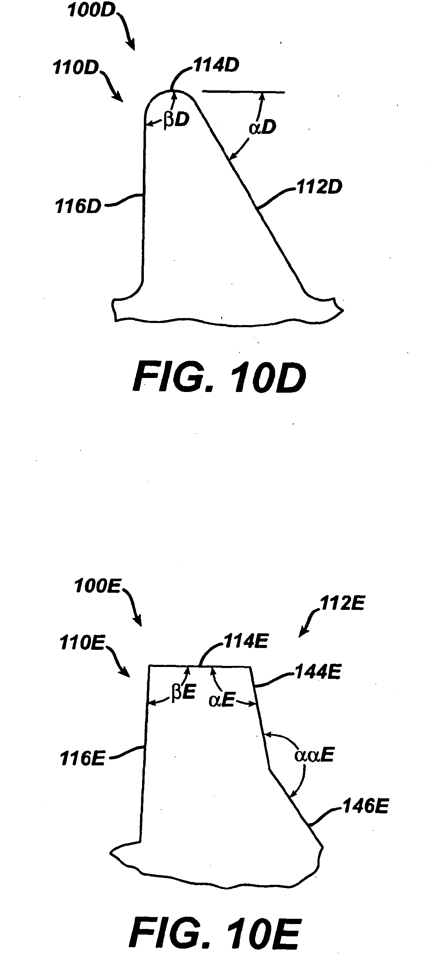

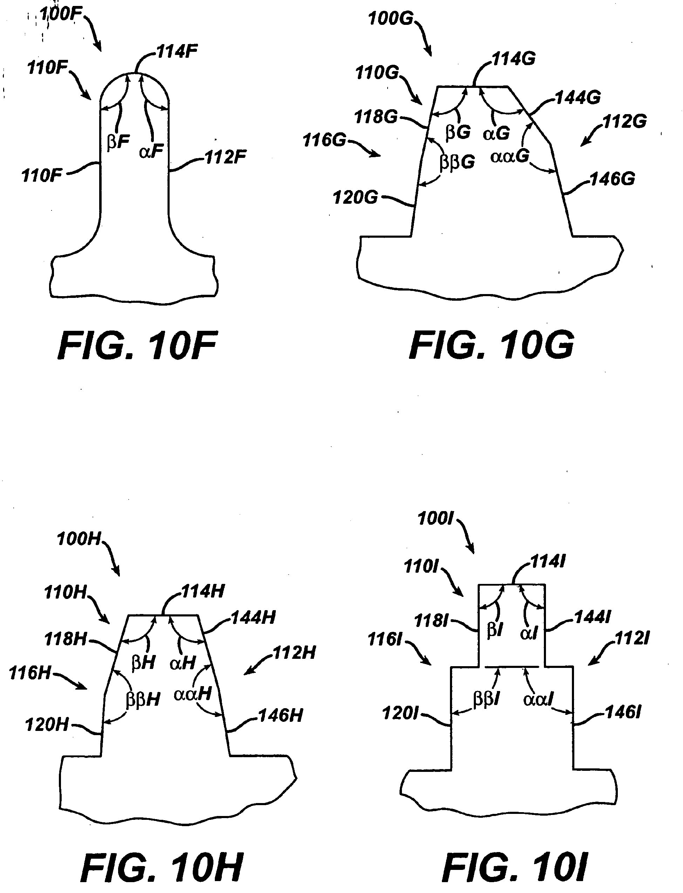

Referring now to FIG. 10, the thread form 110 includes a first flank 112 and a crest 114 adjacent the first flank 112. The thread form 110 also includes a second flank 116 spaced from the first flank 112 and adjacent crest 114. The crest 114 and the first flank 112 form a first angle α therebetween. The crest 114 and the second flank 160 form a second angle P therebetween. The first angle α and the second angle P are different from each other. As shown in FIG. 10, the shank 102 defines a longitudinal axis 118 of the shank 102. The crest 114, as shown in FIG. 10, may be parallel to the longitudinal axis 118.

As is shown in FIG. 10, the first angle α may be acute or less than 90°. The second angle β may as shown in FIG. 10, form a right angle or as shown in FIG. 10, the angle P may be, for example, 90°.

As shown in FIG. 10, the periphery 106 of the shank 102 may further define a second tooth form 120 spaced from the first tooth form 110. The periphery 106 may further define a root 122 positioned between the first tooth form 110 and the second tooth form 120.

As is shown in FIG. 10, the periphery 106 of the shank 102 may include additional tooth forms in addition to the first tooth form 110 and the second tooth form 120. For example and as is shown in FIG. 10, the periphery 106 of the shank 102 may include a third tooth form 124, a fourth tooth form 126, and a fifth tooth form 128. It should be appreciated that other tooth forms, not shown in FIG. 10, may be used. It should be appreciated that each of the tooth forms 110, 120, 124, 126, and 128 may have identical shapes. It should be appreciated that in the alternative, the tooth forms may vary somewhat.

Referring again to FIG. 1, periphery 106 of the shank 102 includes a second portion 130. The second portion 130 of the periphery 106 of the shank 102 as shown in FIG. 1, may define a smooth surface 132. As shown in FIG. 1, the periphery 106 of the shank 102 may be generally cylindrical and may for example, be defined by a diameter, for example, D.

As shown in FIG. 1, the orthopaedic screw 100 of the present invention may further include a head 134. The head 134 may be in the form of, for example, a lip or a collar. The head 134 may extend from the shank 102 of the screw 100 and as shown in FIG. 1, may extend from a second end 136 of the shank 102 opposed to the first mentioned end 104.

As is shown in FIG. 1, the thread form 110 may extend helically around the periphery 106 of the shank 102 for at least two revolutions.

As is shown in FIG. 1, the thread form 110 may extend helically around the periphery shank 102 for several revolutions. For example and is shown in FIG. 1, the screw 100 may as shown in FIG. 1 include a total of, for example, around eight thread forms.

The screw 100 as shown in FIG. 1, is generally cylindrical being defined by diameter D and an overall length L. The shank 102 of the screw 100 includes the first portion 108, which includes the thread form 110 and the second portion 130 having the smooth surface 132. The overall length L of the diameter D is divided into a thread length TL and a smooth length SL. The thread length TL defines the first portion 108 and the smooth length SL defines the second portion 130. The thread length TL may, for example, be a portion of, for example, 20% to 40% of the overall length L of the shank 102. It should be appreciated that the smooth length SL is preferably of sufficient length such that compression of the fracture can occur when the screw 100 is positioned in an intramedullary nail.

The head 134 nay be defined a head thickness HT and a head diameter HD. The head thickness HT and head diameter HD are chosen to be sufficient to provide for a stop medially for the screw 100 when used in an intramedullary nail.

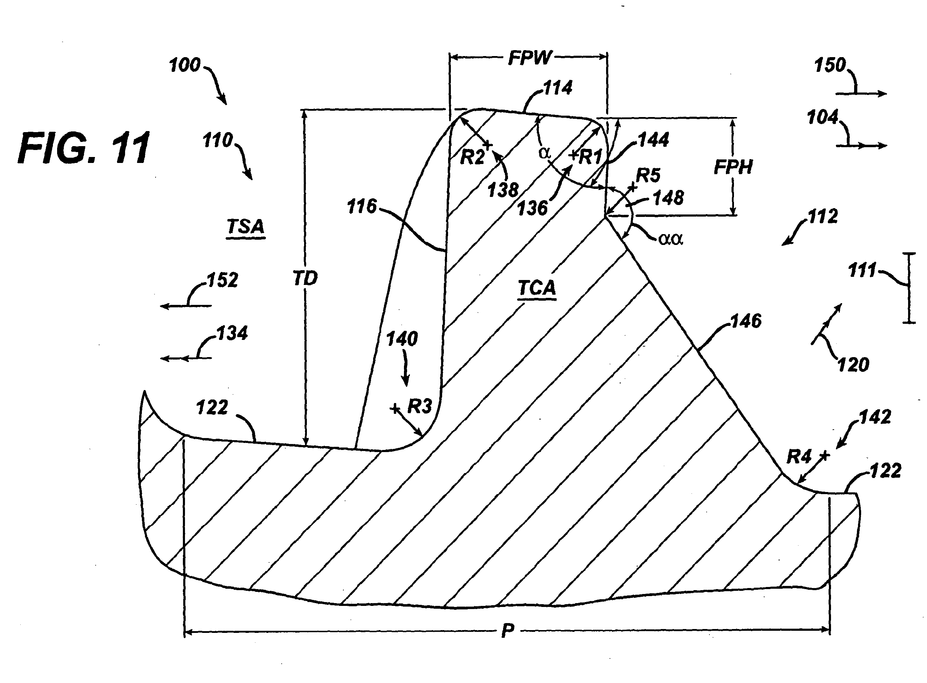

Referring now to FIG. 11, the thread form 110 of the screw 100 is shown in greater detail. A first arcuate feature 136 may, as is shown in FIG. 11, connect the first flank 112 to the crest 114. The arcuate feature 136 may, for example, be in the form of a radius, for example, a radius R1.

A second arcuate feature 138 may connect the second flank 116 to the crest 114. The second arcuate feature 138 like the first arcuate feature 136 may be in the form of, for example, a radius, for example, radius R2. R1 and R2 may be chosen to be large enough to reduce the cutting ability of the thread form 110. By reducing the ability of the thread form 110 to cut cancellous bone, the pull-out forces, the ability to medially migrate, and the cut-out phenomenon of the screw 100 are optimized. The radii R1 and R2 may, for example, be around 1 to 4mm.

Similarly, the thread 110 may include a third arcuate feature 140 positioned between root 122 and the second flank 116. The third arcuate feature 140 may be in the form of a radius, for example radius R3. The thread form 110 may also include a fourth arcuate feature 142 positioned between root 122 and first flank 112. The fourth arcuate feature 142 may be in the form of, for example, a radius R4.

The radii R3 and R4 may be chosen to minimize stress risers for the screw 100 and to optimize the thread cutting ability of the screw 100. For example, the radii R3 and R4 may be, for example, 2 to 5 mm.

While the first flank 112 may, it should be appreciated, be flat or linear, as shown in FIG. 11, the first flank may include a first portion 144 extending from the crest 114 and a second portion 146 extending from the first portion 144. The first portion 144 and the second portion 146 may, as is shown in FIG. 11, have a different orientation. For example and is shown in FIG. 11, the first portion 144 may form an angle α of, for example, 90° with the crest 114. The second portion 146 may form an obtuse angle αα with the first portion 144. The obtuse angle may be approximately 140°.

The first arcuate portion 144 and the second arcuate portion 146 may define an arcuate feature 148 therebetween. The arcuate feature 148 may be in the form of, for example, a radius R5. The radius R5 may be chosen to minimize stress risers and to provide for the proper thread cutting. For example, radius R5, for example, 1 to 4 mm.

The distance between adjacent thread forms, for example, first thread form 110 and second thread form 120 may be defined by a dimension, a pitch P. The pitch P may be chosen relative to other factors such as the diameter D of the screw 100. The dimension of the pitch P may be selected to provide for increasing thread pull-out forces and reducing medial migration incidents.

The crest 114 and the root 122 define a thread depth TD. The thread depth TD is selected to optimize the work required to rotate the screw 100 through cancellous bone and to minimize problems with thread pull-out and medial migration.

The first portion 114 defines a first portion height FPH. The first portion height FPH is selected as a portion of the thread depth TD to compromise between torque required to thread the screw 100 into cancellous bone and to minimize medial migration of the screw 100. The first portion height FPH is, for example, a percentage of the thread depth TD. For example, the first portion height FPH may be, for example, 20 to 40% of the thread depth TD. For example, the first portion height FPH may be for example, around ¼ of the thread depth TD.

The thread form 110 is chosen to optimize the strength of the thread form 110, the ability of the thread form 110 to cut threads and to avoid medial migration and to preserve the strength remaining in the portion of the cancellous bone between the adjacent thread forms 110.

For example and is shown in FIG. 11, the thread form 110 defines a thread form cross-sectional area TCA bounded by, as shown in FIG. 11, the second flank 116, the crest 114, and the first portion 110 and the second portion 116 of the first flank 112. Between adjacent thread forms a thread spacing area TSA is formed between the first flank 112 of the first thread form 110 and the second flank of the second thread form 120. The relative size of the thread cross-sectional area TCA and the thread spacing area TSA is chosen to minimize medial migration while providing for sufficient thread pull-out force.

The thread spacing area TSA establishes the area of cancellous bone that must be displaced by the screw 100 during pull-out. For example and is shown in FIG. 11, the ratio of the thread cross-sectional area to the thread spacing area may be, for example, from around 30% to around 50%. For example, the thread cross-sectional may be around 40% of the total cross-sectional area including the thread cross-sectional area and the tooth spacing cross-sectional area.

First portion 144 of the first flank 112, requires that the cancellous bone in the femur be condensed when advancing the screw 100 in the direction of arrow 150 toward end 104. Thus the first portion 144 serves to limit medial migration of the screw 100 in the direction of arrow 150. Similarly, the second flank 116 causes the cancellous bone in the femur to be compressed when the screw 100 is advanced in the direction of arrow 152 toward head 134. Thus, the second flank 116 serves to prevent or establish for the amount of pull-out required to move the screw 100 in the direction of arrow 152.

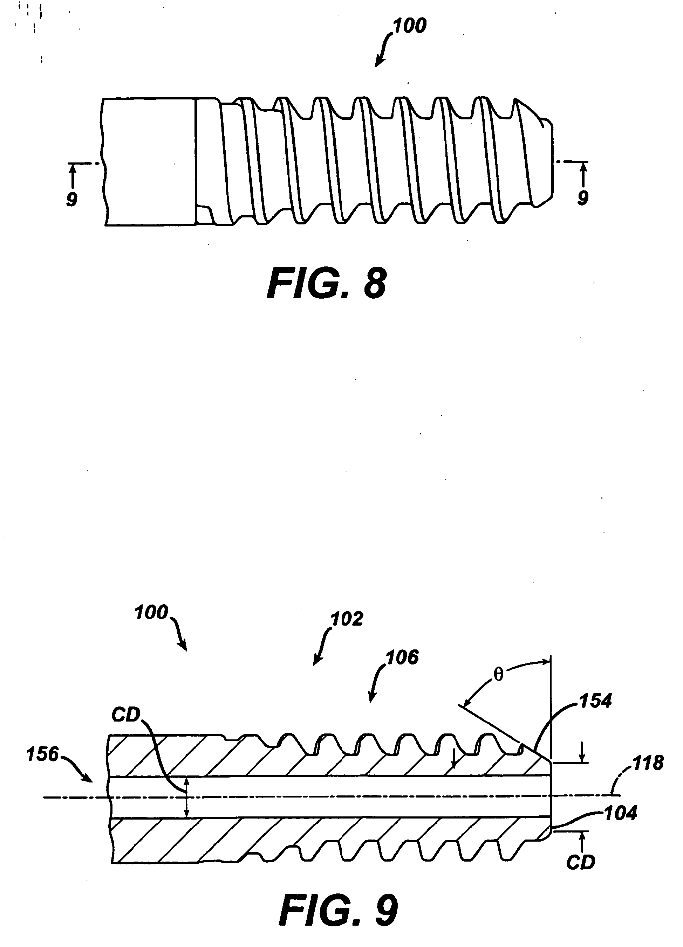

Referring now to FIGS. 8-9, the screw 100 is shown in greater detail. As can be seen in FIGS. 8-9 each of the adjacent thread forms 110 have substantially the same shape. It should be appreciated however, to assist in the installing of the screw 100 into cancellous bone a chamfer 154 is formed on periphery 106 of the shank 102 of the screw 100. The chamfer 154 may, is as shown FIGS. 8-9 be defined by a chamfer angle θ and by chamfer diameter CD. The chamfer diameter CD and chamfer angle θ to provide for proper thread torque and to minimize medial migration.

Referring now to FIGS. 10A-10I, alternative thread forms are shown. These alternative thread forms are believed to be effective to reduce medial migration and cut-out of the lag screws.

Referring now to FIG. 10A, tooth form 110A of screw 100A is shown. The tooth form 100A includes a crest 114A from which first flank 112A extends at an angle αA. The second flank 116A extends from crest 114A at an angle βA that is obtuse. The angle βA is greater than 90°.

Referring now to FIG. 10B, another tooth form in the form of tooth form 110B is shown for screw 100B. The tooth form 110B includes a crest 114B. First flank 112B extends from crest 114B at angle αB. The first flank 112B includes a first portion 144B and a second portion 146B. The second portion 146B extend from first portion 144B at obtuse angle ααB. The tooth form 110B further includes a second flank 116B extending from crest 114B at an angle βB.

Referring now to FIG. 10C, yet another tooth form is in the form of tooth form 110C for screw 100C. The tooth form 110C includes a crest 114C from which first flank 112C and second flank 116C extend. The first flank 112C and the second flank 116C are normal or perpendicular to the crest 114C and therefore parallel to each other. The angle BC and αC are thus 90°.

Referring now to FIG. 10D, another tooth form in the form of tooth form 110D for screw 100D is shown. The tooth form 110D includes a crest 114D that is arcuate. A first flank 112 D extends from the crest 114D at an angle αD and a second flank 116D extends perpendicularly from the crest 114D at angle BD.

Referring now to FIG. 10E, yet another tooth form in the form of tooth form 1120E for screw 110E is shown. The tooth form 110E includes a crest a 114E from which first flank 112E and second flank 116E extend. The first flank 112E includes a first portion 144E formed, angle αE with crest 114E and a second portion 146E forming angle ααE with first portion 144E. The second flank 116E extends perpendicularly or normally from the crest 114E.

Referring now to FIG. 10F, yet another tooth form in the form of tooth form 110F for screw 100F is shown. The tooth form 100F includes a crest 114F which is arcuate. Extending from the crest 114F are first flank 112F and second flank 116F. The first flank 112F and the second flank 116F are normal or perpendicular to the crest 114F and therefore parallel to each other. The angles F and αF are both 90°.

Referring now to FIG. 10G, yet another tooth form in the form of, tooth form 110G is shown. The tooth form 110G is for use with screw 100G. The tooth form 110G includes a crest 114G from which first flank 112 and second flank 116 extend. The first flank 112G includes a first portion 144G and a second portion 146G. The second flank 146G includes a first portion 118G and a second portion 120G. The first flank 112G and the second flank 116G as shown in FIG. 10G are not symmetrical. Thus, angles αG and BG are different and angles ααG and BBG are different.

Referring now to FIG. 10H, another tooth form in the form of tooth form 110H for use with screw 100H is shown. The tooth form 110H includes a crest 114H from which a first flank 112H and a second flank 116H extend. The first flank 112H includes a first portion 144H and a second portion 146H. The second flank 116H includes a first portion 118H and a second portion 120H. The tooth form 110H of FIG. 10H, is different than the other tooth forms in that the first flank 112 and the second flank 116 are symmetrical about the crest 114H. Angles αH and BH are the same and angles ββH and ααH are the same.

Referring now to FIG. 10I, yet another tooth form in the form of, tooth form 110I for use with screw 100I is shown. The tooth form 110I includes a crest 114I from which a first flank 112I and a second flank 116I extend. The first flank 112I includes a first portion 144I and a second portion 146I. The second flank 116I includes a first portion 118I and a second portion 120I. The portions 144, 146, 118, and 120 are shown in FIG. 10I are normal or perpendicular to the crest 114I or consequently are all parallel to each other. Angles αI, BI, ααI and ββI are all 90°.

Referring now to FIG. 9, the screw 100 may include a central opening or cannula 156 positioned concentric with longitudinal axis 118 of the screw 100. The cannula 156 may be defined by a cannula diameter CD. The diameter CD is selected for fitting with a wire that may be used with the installation of the screw 100 into the cancellous bone of the femur 2.

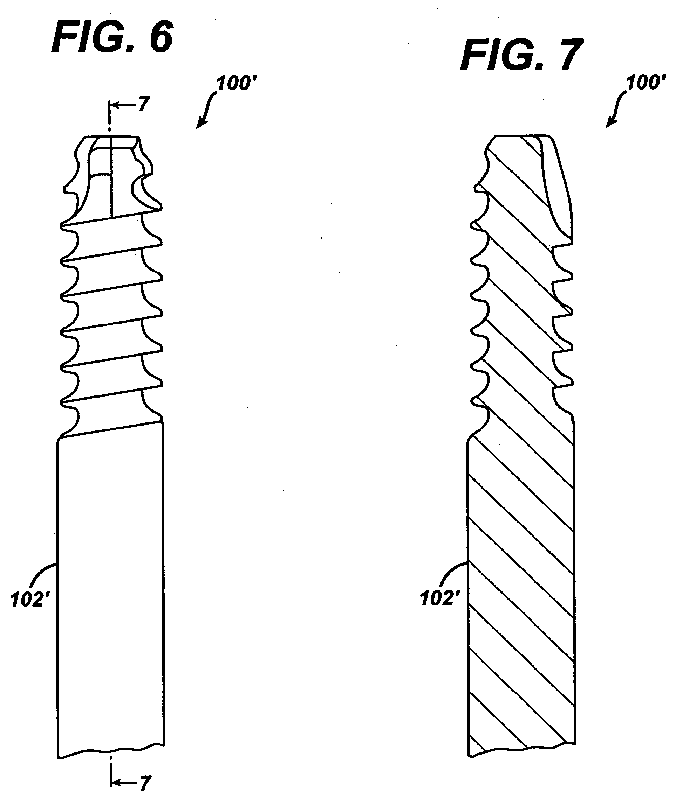

Referring now to FIGS. 6 and 7, an alternate embodiment of the present invention is shown as orthopaedic screw 100. The orthopaedic screw 100 is similar to the orthopaedic screw 100 of FIGS. 1 and 8-11 except that the screw 100 includes a shank 102 prime that is solid or not cannulated.

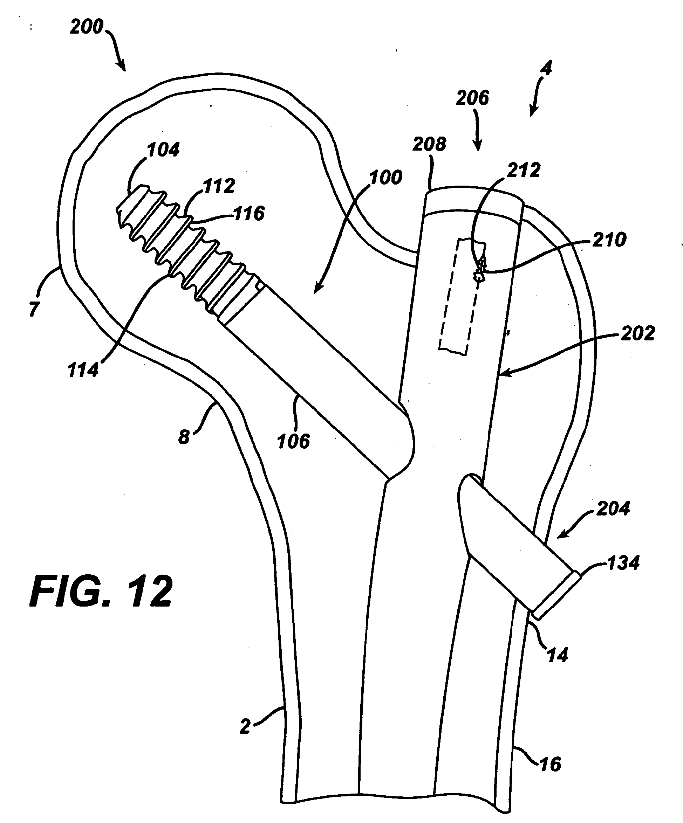

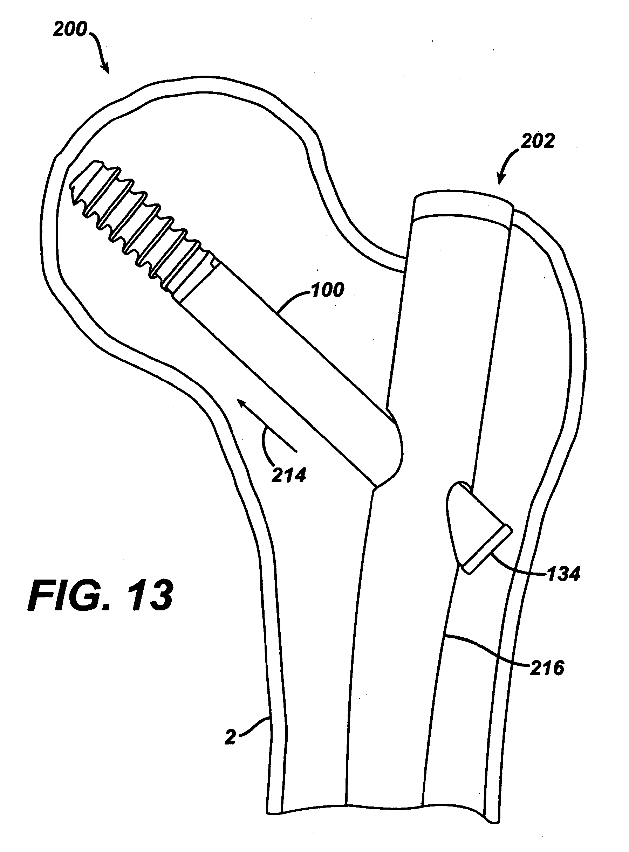

Referring now to FIGS. 12 and 13, another embodiment of the present invention is shown as trochanteric nail assembly 200. The trochanteric or intramedullary nail assembly 200 is designed for use in a medullary canal for of a long bone 2. The nail assembly 200 includes a nail 202 for positioning for at least partially of the medullary canal 4. The nail 202 includes an aperture or opening 204 through the nail 202.

The nail assembly 200 further includes a screw, for example, screw 100 of FIGS. 1 and 8-11. The screw 100 is fittably positioned in the aperture 204 of the nail 202. The screw 100 includes the shank 102 defining end 104 and periphery 106. A portion of the periphery 106 defines thread form 110. The thread form 110 includes first flank 112, crest 114 adjacent to the first flank 112, and second flank 116 spaced from the first flank 112 adjacent the crest 114. The crest 114 and the first flank 112 form a first angle therebetween. The crest 114 and the second flank form a second angle therebetween. The first angle and the second angle are different from each other.

As shown in FIGS. 12 and 13, the orthopaedic screw 100 is utilized with nail 202 in the form of a trochanteric nail. It should be appreciated that the screw 100 may be used with an intramedullary nail, for example, a femoral nail, tibial nail, antegrade nail, retrograde nail, or a universal nail capable for use for various indications.

As shown in FIG. 12, the orthopaedic screw 100 is fitted into neck 8 and head 7 of the long bone 2. The long bone 2 may as is shown in FIG. 12 be, for example, a femur. Intramedullary nail 202 may be any suitable nail and may, as shown in FIG. 12, be canulated or define a longitudinal opening 206 extending the length of the nail 202. The intramedullary nail may also include a cap 208 threadably secured to the intramedullary nail 202 by external threads 210 formed on the cap which mate with internal threads 212 formed in the opening 206 of the intramedullary nail 202.

As shown in FIG. 12, a solitary or single screw may be utilized. The screw 100 as shown in FIG. 12 may include the head 134. The head 134, as is shown in FIG. 12, preferably rests against internal wall 14 of the cordical bone 16 of the long bone 2.

Referring now to FIG. 13, the nail assembly 200 is shown installed on long bone 2 with the screw 100 advancing in the direction of arrow 214 or advancing medially. While such medial migration should be unlikely with the thread form 110 of the screw 100 of the present invention, it should be appreciated that the medial migration in the direction of arrow 214 will be physically limited by the seating of the head 134 against outer periphery 216 of the trochanteric nail 202.

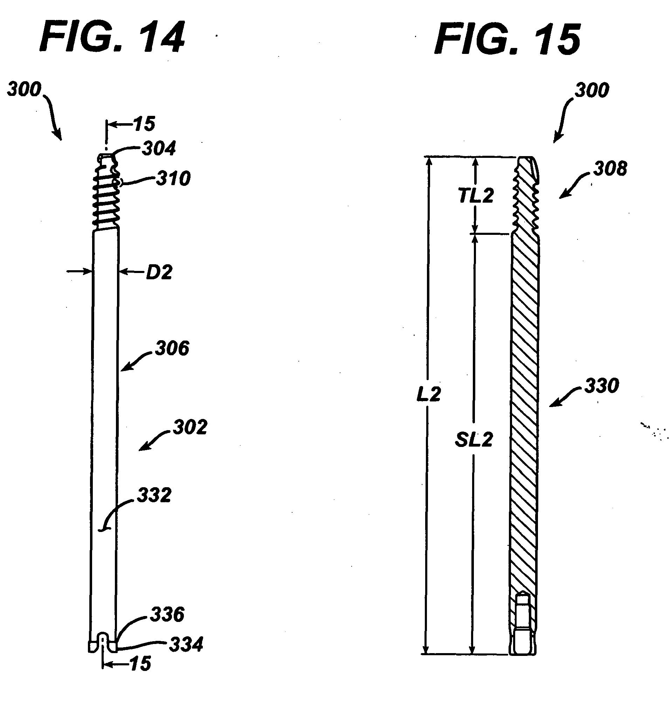

Referring now to FIGS. 14, 15, and 16 an anti-rotation screw 300 for use with the present invention is shown. The anti-rotation screw 300 includes a shank 302 which as is shown in FIG. 14 and 15, is generally cylindrical. The shank 302 unlike the shank 102 of the screw 100 of FIG. 1 is not canulated.

The screw 300 further includes a periphery 306 which defines a first portion 302 which includes a smooth surface 332 and a second portion 308 which defines a thread form 310. A head 334 extends from second end 336 of the shank 302 opposed to the first end 304 of the shank 302. The shank 302 may, as shown in FIGS. 14 and 15, have a diameter D2 and a length L2. The length L2 is divided through a thread-length TL2 in the first portion 308 and a smooth length SL2 in the second portion 330 of the shank 302.

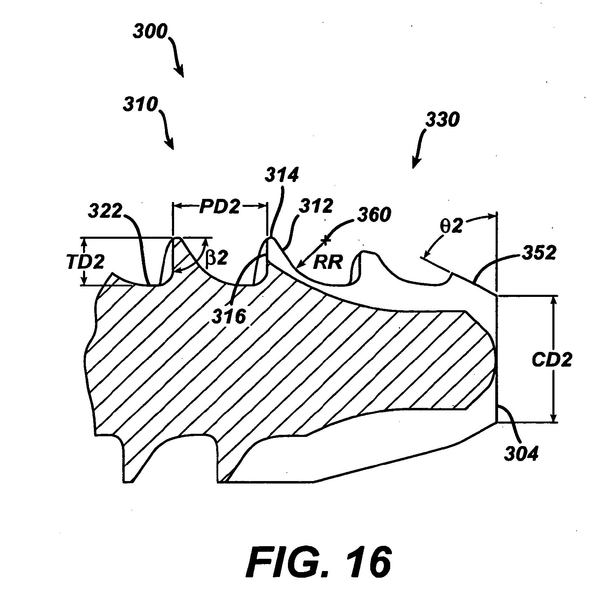

Referring now to FIG. 16, the thread-form 310 is shown in greater detail. The thread-form 310 includes a crest 314 from which first flank 312 and second flank 316 extend. The first flank 312 is arcuate and, as is shown in FIG. 16, is defined by a radius RR extending from origin 360. The second flank 316 forms an angle B2 with respect to the crest 314. The angle b2 may, as shown in FIG. 16, be approximately 90°.

The thread-form 310 may further define a root 322. The root 322 and the crest 314 define a thread-depth TD-2. Adjacent thread-forms 310 define a thread-pitch P2. The pitch P2 and the thread-depth TD2 are selected to provide for proper insertion torque and to maximize the required pull-out force and to minimize medial migration. The screw 300 further includes a chamfer 352 extending from first end 304 of the screw 300. The chamfer 352 may be defined by chamfer diameter CD2 and chamfer angle θ2. Angle 02 and the diameter CD2 are chosen to provide for reasonable insertion torque and to minimize medial migration.

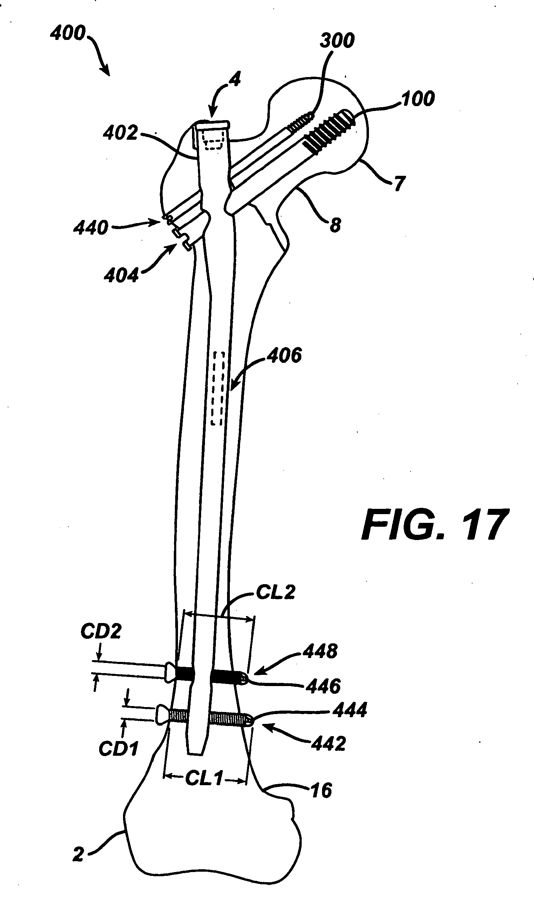

Referring now to FIGS. 17-20, another embodiment of the present invention is shown as intramedullary nail assembly or trochanteric nail assembly 400. The nail assembly 400 is similar to the nail assembly 200 of FIGS. 12 and 13, except that the nail assembly 400 further includes anti-rotation screw 300 in addition to the lag screw 100.

The nail assembly 400 includes the lag screw 100, the anti-rotation screw 300, and a trochanteric nail 402. The trochanteric nail 402 is fitted into intramedullary canal 4 of the long bone 2. The nail 402 includes a first opening 404 for slidable passage of the lag screw 100 as well as a parallel spaced apart second opening 440 for slidable fitting with the anti-rotation screw 300.

Nail 302 may also include a longitudinal cannula or opening 406. The nail 402 may further include a first distal opening 442 for receiving a first cortical screw 444 for engagement with the cortical bone 16 of the long bone 2. The first cortical screw 444 may be defined by a length CL2 and a diameter CD 1. The nail 402 may use a solitary cortical screw 444 but may, as is shown in FIG. 17, include a second cortical screw 446 which is fitted to second distal transverse opening 448 formed in the nail 402. The second cortical screw 446 may be defined by a length CL2 and a diameter CD2. The second cortical screw 446 engages the cortical bone 16 of the long bone 2.

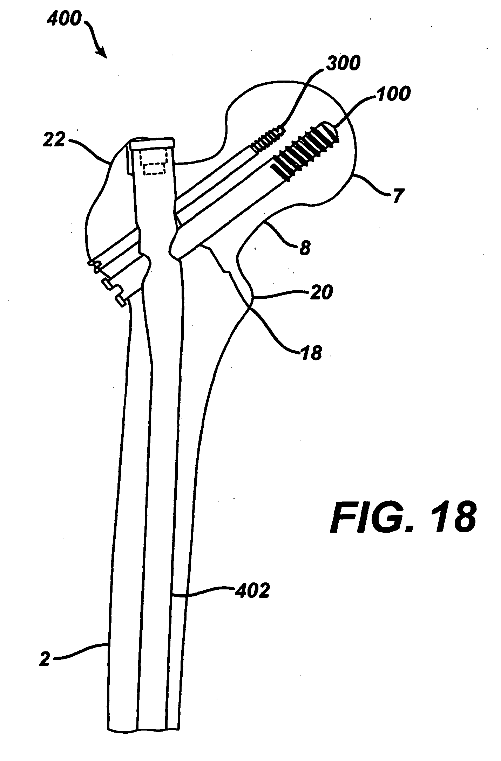

Referring to FIGS. 17 and 18, the lag screw 100 and the anti-rotation screw 300 are adapted to extend into neck 8 and head 7 of, for example and as shown in FIGS. 17 and 18, a long bone 2 in the form of a femur.

As is shown in FIG. 18, the nail assembly 400 of FIGS. 17 to 20 is adapted for a inter-trochanteric fracture 18 extending from, for example, the lesser trochanter 20 to the greater trochanter 22.

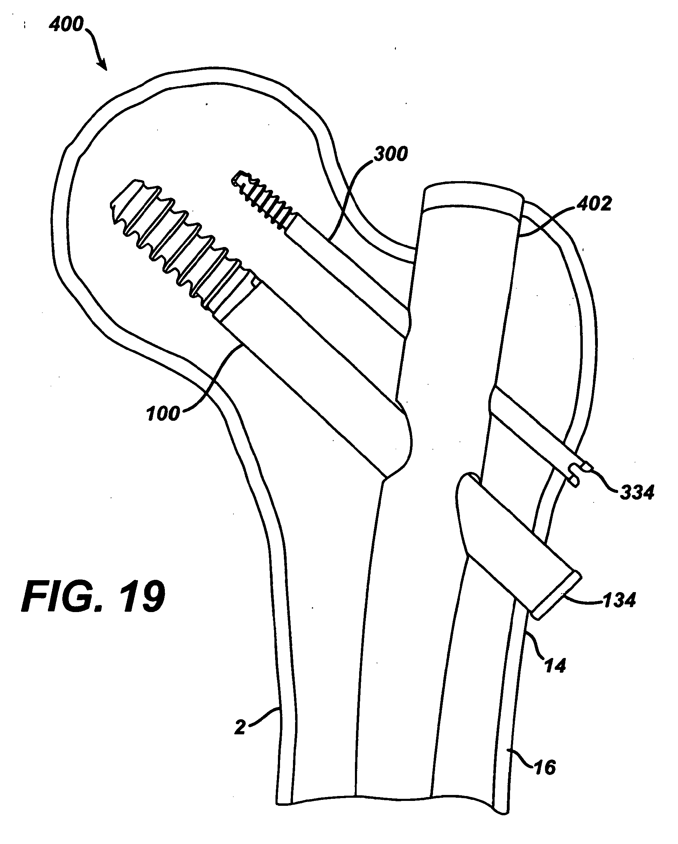

Referring now to FIG. 19, the lag screw 100 is positioned in the nail 402 such that head 134 of the lag screw 100 is seated against periphery 14 of the cortical bone 16 of the femur 2. Similarly, the anti-rotation screw 300 is positioned in the nail 302 such that the head 334 of the anti-rotation screw 300 seats against periphery 14 of the cortical bone 16 of the femur 2.

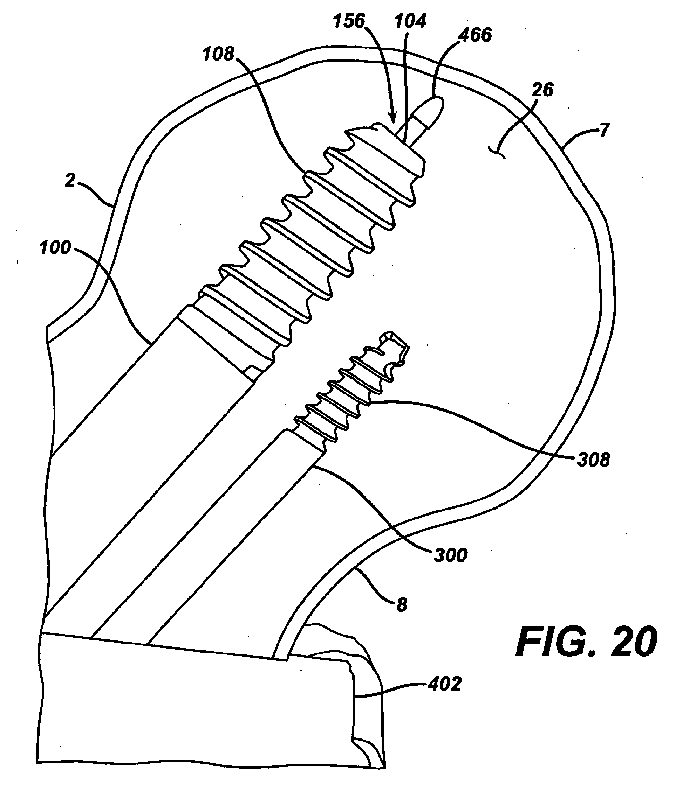

Referring now to FIG. 20, the first portion or threaded portion 108 of the lag screw 100 and the first portion or threaded portion 308 of the anti-rotation screw 300 are shown positioned in cancellous bone 26 of the head 7 of the femur 2. As shown in FIG. 20, a wire 466 is shown extending from end 104 of the lag screw 100. The wire 466 slidably fits into the longitudinal opening or cannula 156 of the nail 100.

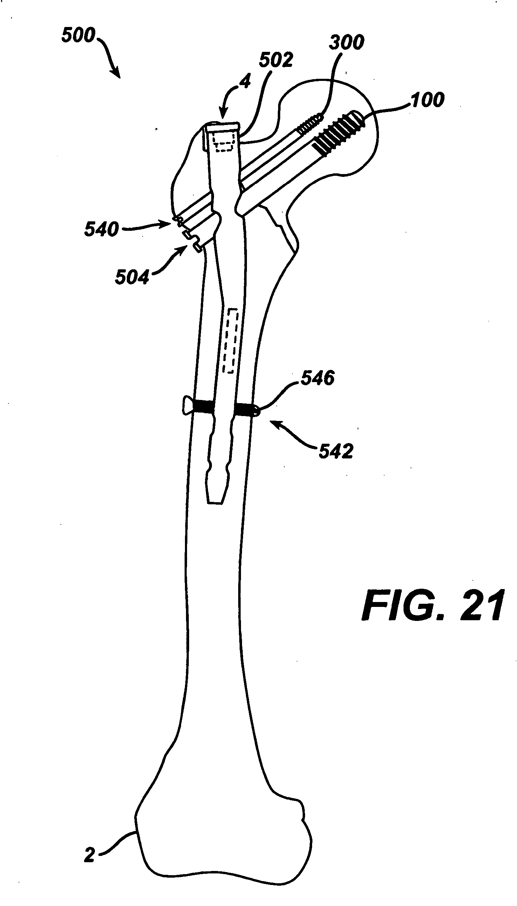

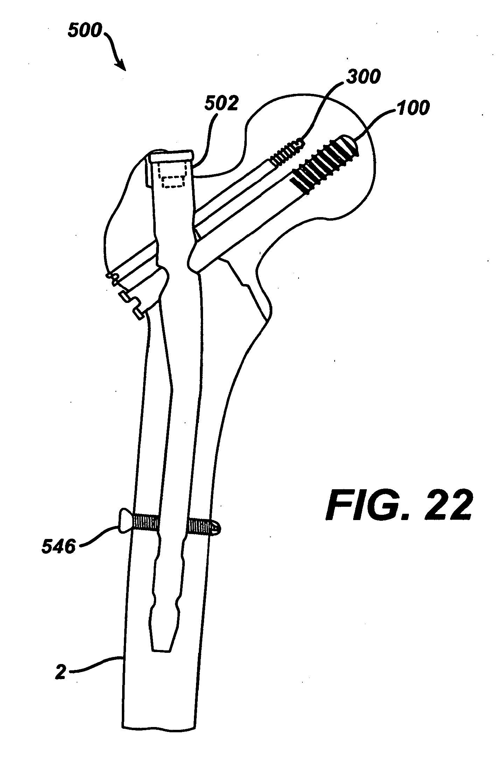

Referring now to FIGS. 21 and 22, yet another embodiment of the present invention is shown as trochanteric nail assembly or intramedullary nail assembly 500. The intramedullary nail assembly 500 is similar to the intramedullary nail assembly 400 of FIGS. 17-20, except that the intramedullary nail assembly 500 of FIGS. 21 and 22 includes a trochanteric nail 502 which is shorter than the trochanteric nail 402 of the nail assembly 400 of FIGS. 17-20.

The nail assembly 500 includes nail 502 fitted into canal 4 of the femur 2. The nail 502 includes a first transverse opening 504 for slidable fitting with the lag screw 100. Similarly, a second transverse opening 504 is positioned parallel in space from the first transverse opening 504. The second transverse opening 540 is adapted for slidably fitting of the anti-rotation screw 300. The nail 502 further includes a distal opening 542 for receiving distal screw 546 similar to distal screw 446 of the nail assembly 400 of FIGS. 17 to 20.

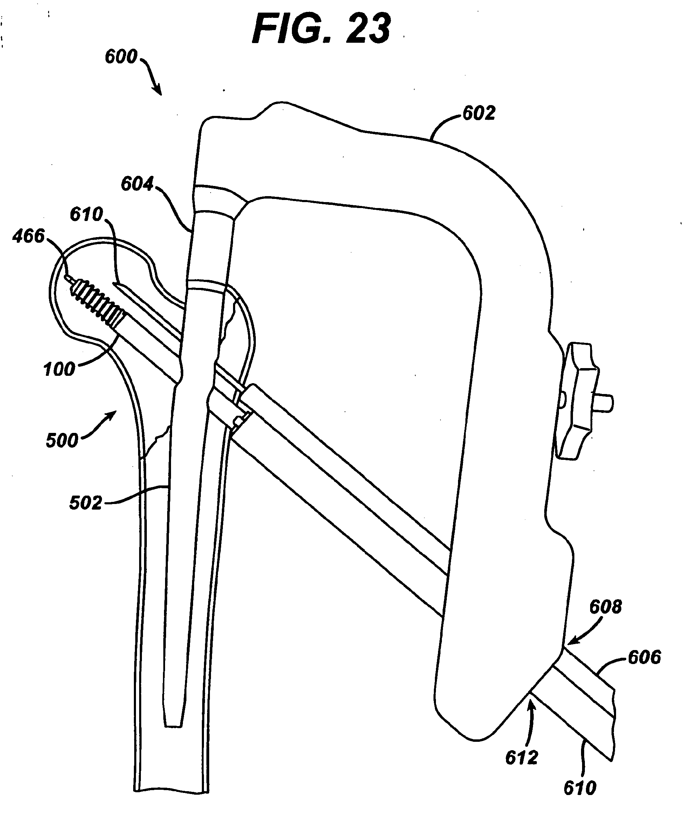

Referring now to FIGS. 23 to 26, an instrument 600 is shown for use with the nail assembly 500 of FIGS. 21 and 22. The instrument 600 includes a body 602. The body 602 may be made of a radiolucent material, for example, a carbon fiber material, to assist in fluoroscopic procedures in installing the nail assembly 502. The body 602 may define a series of openings for guiding the screws through the femur 2 and into the nail 502.

The instrument 600 may further include a connector or adapter 604 for cooperating with the nail 500 and for locking and orienting the nail 500 to the instrument 600. The adapter 604 may include features which cooperate with, for example, the end and the longitudinal opening of the nail 500. The instrument 600, as shown in FIG. 23, may include a first sheath 606 which matingly fits with first sheath opening 608 formed in the body 602 of the instrument 600. The first sheath 606 is utilized to guide an anti-rotation drill 610 into the femur 2. The instrument 600 may further include a second sheath 610 which is fitted through second opening 612 in the body 602 of the adapter 600. The second sheath 600 is used to guide the guide-wire 446 into the femur 2. The sheath 610 and the guide-wire 466 are used to guide the lag screw 300 into the nail 502 and into the femur 2.

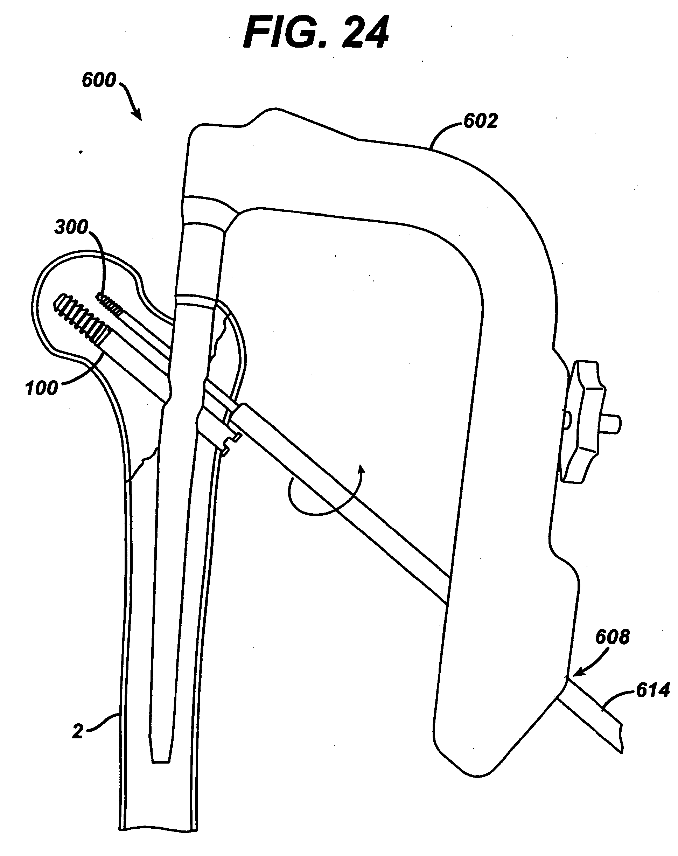

Referring now to FIG. 24, the instrument 600 further includes a third sheath 614 which is fitted into the first opening 608 in the body 602 of the instrument 600. The third sheath 614 is used to guide the anti-rotation screw 300 through the nail 502 and into the femur 2.

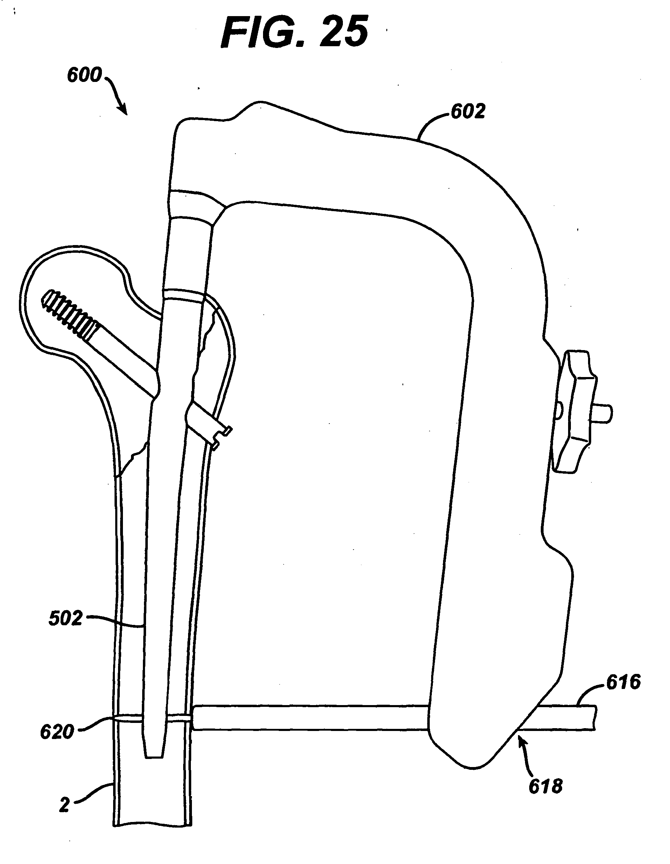

Referring now to FIG. 25, the instrument 600 further includes a fourth sheath 616 which is fitted into third opening 618 formed in the body 602 of the instrument 600. The sheath 616 is utilized to guide a distal screw drill 620 into the femur 2 and the nail 502.

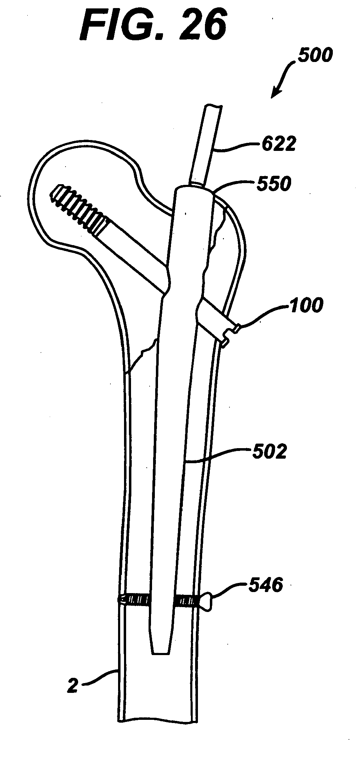

Referring now to FIG. 26, the nail assembly 500 is shown positioned in femur 2 with the distal screw 546 and the lag screw 100 in position in the nail 502. A screwdriver 622 is shown installing the cap 550 onto the nail 502.

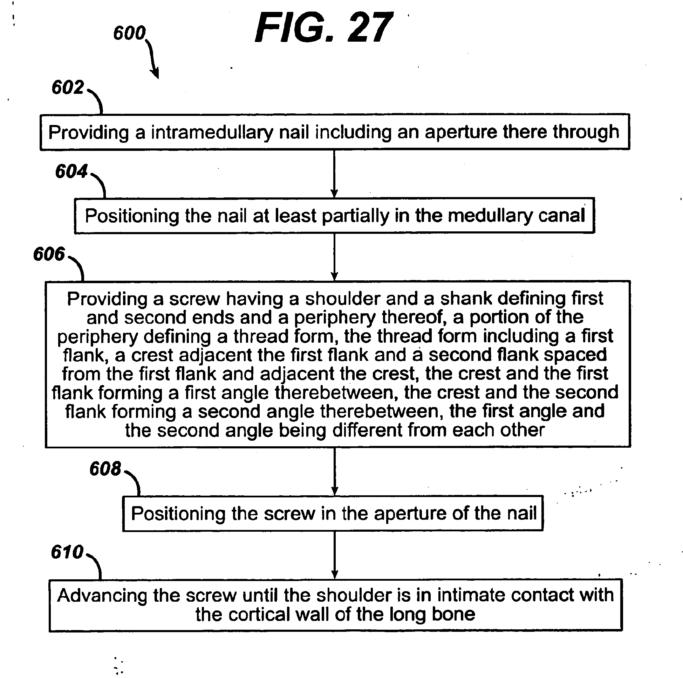

Referring now to FIG. 27, yet another embodiment is shown as method 600 for performing trauma surgery. The method 600 includes a first step of providing an intramedullary nail, including an aperture in the nail. The method 600 includes a second step 604 of positioning the nail at least partially in the medullary canal. The method 600 further includes a third step 606 of providing the screw having a shoulder and a shank defining first and second ends. The screw includes a periphery thereof with a portion of the periphery defining a thread-form. The thread-form includes a first flank, a crest adjacent to the first flank, and a second flank spaced from the first flank and adjacent the crest. The crest and the first flank form a first angle between each other. The crest and the second flank form a second angle between each other. The first angle and the second angle are different from each other. The method 600 further includes a fourth step 608 of positioning the screw in the aperture of the nail.

The method 600 further includes a fifth step 610 of advancing the screw until the solider has into intimate contact with the cordical wall of the long bone.

## Claims

1. An orthopaedic screw comprising a shank (102) defining an end (104) and a periphery (106) thereof, a portion (108) of the periphery defining a thread form (110), the thread form including a first flank (112), a crest (114) adjacent the first flank and a second flank (116) spaced from the first flank and adjacent the crest, the crest and the first flank forming a first angle (α) therebetween, the crest and the second flank forming a second angle (β) therebetween, the first angle and the second angle being different from each other, in which the first flank is positioned adjacent the end (104), **characterised in that** the first flank defines a first portion (144) extending from the crest and a second portion (146) extending from the first portion, the first portion and the second portion having different orientations, in which the second flank is positioned opposed to the end (104).

2. The orthopaedic screw as in claim 1, in which said shank (102) defines a longitudinal axis (118) thereof; and in which the crest (114) is parallel to the longitudinal axis.

3. The orthopaedic screw as in claim 1, in which the angle (α) between the first flank (112) and the crest (114) is at least about 90°.

4. The orthopaedic screw as in claim 1, in which a radius is formed at least one of between the crest (114) and the first flank (112) and between the crest (114) and the second flank (116).

5. The orthopaedic screw as in claim 1, in which the periphery (106) of the shank (102) further defines a second tooth form (120) spaced from the first mentioned tooth form (110); and in which the periphery further defines a root (122) positioned between the first mentioned tooth form and the second tooth form.

6. The orthopaedic screw as in claim 5, in which a radius is formed at least one of between the root (122) and the first mentioned tooth form (110) and between the root and the second tooth form (120).

7. The orthopaedic screw as in claim 1, in which an arcuate feature (136), preferably in the form of a radius, connects at least one of the first flank (112) to the crest (114) and the second flank (116) to the crest (114).

8. The orthopaedic screw as in claim 1, in which the angle between the first portion (144) of said first flank (112) and said crest (114) is at least about 90°.

9. The orthopaedic screw as in claim 1, in which a second portion (130) of the periphery (106) of said shank (102) defines a smooth surface (132).

10. The orthopaedic screw as in claim 1, in which the periphery (106) of said shank (102) is generally cylindrical.

11. The orthopaedic screw as in claim 1, which includes a head (134) extending from said shank (102) and opposed to the first mentioned end (104).

12. The orthopaedic screw as in claim 1, in which the thread form (110) extends helically around the periphery (106) of said shank (102) for at least two revolutions.

13. An intramedullary nail assembly (200) for use in a medullary canal of a long bone (2), said assembly comprising:
a nail (202) for positioning at least partially in the medullary canal, said nail including an aperture (204) therethrough; and
a screw (100) as claimed in any one of claims 1 to 12.

## Patentansprüche

1. Orthopädische Schraube, die einen Schaft (102) aufweist, welcher ein Ende (104) und eine Begrenzungsfläche (106) dieser abgrenzt, wobei ein Abschnitt (108) der Begrenzungsfläche eine Gewindeform (110) abgrenzt, die Gewindeform eine erste Flanke (112), einen an die erste Flanke angrenzenden Gewindescheitel (114) und eine zweite von der ersten Flanke beabstandete und an den Gewindescheitel angrenzende Flanke (116) umfasst, wobei der Gewindescheitel und die erste Flanke einen ersten Winkel (α) zwischen sich ausbilden, der Gewindescheitel und die zweite Flanke einen zweiten Winkel (β) zwischen sich ausbilden, und wobei sich der erste Winkel und der zweite Winkel voneinander unterscheiden und die erste Flanke neben dem Ende (104) angeordnet ist, **dadurch gekennzeichnet, dass** die erste Flanke einen ersten Abschnitt (144) abgrenzt, der sich von dem Gewindescheitel erstreckt, und einen zweiten Abschnitt (146), der sich von dem ersten Abschnitt erstreckt, wobei der erste Abschnitt und der zweite Abschnitt verschiedene Ausrichtungen aufweisen, bei denen die zweite Flanke gegenüber dem Ende (104) angeordnet ist.

2. Orthopädische Schraube nach Anspruch 1, bei der der Schaft (102) eine längs laufende Achse (118) dieser festlegt; und bei der der Gewindescheitel (114) parallel zu der längs laufenden Achse vorliegt.

3. Orthopädische Schraube nach Anspruch 1, bei der der Winkel (α) zwischen der ersten Flanke (112) und dem Gewindescheitel (114) zumindest ungefähr 90° beträgt.

4. Orthopädische Schraube nach Anspruch 1, bei der ein Rundungsradius zumindest zwischen einem von Gewindescheitel (114) und der ersten Flanke (112), und zwischen dem Gewindescheitel (114) und der zweiten Flanke (116) ausgebildet ist.

5. Orthopädische Schraube nach Anspruch 1, bei der die Begrenzungsfläche (106) des Schaftes (102) darüber hinaus eine zweite Zahnform (120) abgrenzt, welche von der zuerst genannten Zahnform (110) beabstandet ist; und bei der die Begrenzungsfläche darüber hinaus einen zwischen der zuerst genannten Zahnform und der zweiten Zahnform angeordneten Gewindegrund (122) abgrenzt.

6. Orthopädische Schraube nach Anspruch 5, bei der ein Rundungsradius zumindest zwischen einem von dem Gewindegrund (122) und der zuerst genannten Zahnform (110), und zwischen dem Gewindegrund und der zweiten Zahnform (120) ausgebildet ist.

7. Orthopädische Schraube nach Anspruch 1, bei der ein bogenförmiges Element (136), vorzugsweise mit der Form eines Rundungsradius, zumindest eine von der ersten Flanke (112) mit dem Gewindescheitel (114), und der zweiten Flanke (116) mit dem Gewindescheitel (114) verbindet.

8. Orthopädische Schraube nach Anspruch 1, bei der der Winkel zwischen dem ersten Abschnitt (144) der ersten Flanke (112) und dem Gewindescheitel (114) zumindest ungefähr 90° ist.

9. Orthopädische Schraube nach Anspruch 1, bei der ein zweiter Abschnitt (130) der Begrenzungsfläche (106) des Schafts (112) eine ebene Fläche (132) abgrenzt.

10. Orthopädische Schraube nach Anspruch 1, bei der die Begrenzungsfläche (106) des Schafts (102) im wesentlichen zylindrisch ist.

11. Orthopädische Schraube nach Anspruch 1, die einen Kopf (134) beinhaltet, welcher von dem Schaft (112) absteht und dem zuerst erwähnten Ende (104) gegenüberliegt.

12. Orthopädische Schraube nach Anspruch 1, bei der sich die Gewindeform (110) spiralförmig um die Begrenzungsfläche (106) des Schafts (102) für zumindest zwei Umläufe erstreckt.

13. Marknagelanordnung (200) zur Verwendung in einem Markkanal eines langen Knochens (2), wobei diese Anordnung aufweist:
einen Nagel (202) zur Platzierung zumindest teilweise im Markkanal, wobei der Nagel einen Durchlass (204) durch sich einschließt; und
eine Schraube (100), wie in irgendeinem der Ansprüche 1 bis 12 beansprucht.

## Revendications

1. Vis orthopédique comprenant une tige (102) définissant une extrémité (104) et une périphérie de celle-ci (106), une partie (108) de la périphérie définissant une forme filetée (110), la forme filetée comprenant une première face (112), une crête (114) adjacente à la première face et une seconde face (116) espacée de la première face et adjacente à la crête, la crête et la première face formant un premier angle (α) entre elles, la crête et la seconde face formant un second angle (β) entre elles, le premier angle et le second angle étant différents l'un de l'autre, la première face étant placée au niveau adjacent à l'extrémité (104), **caractérisée en ce que** la première face définit une première partie (144) s'étendant de la crête et une seconde partie (146) s'étendant de la première partie, la première partie et la seconde partie ayant des orientations différentes, la seconde face étant placée au niveau opposé à l'extrémité (104).

2. Vis orthopédique selon la revendication 1, dans laquelle ladite tige (102) définit un axe longitudinal (118) de celle-ci ; et dans laquelle la crête (114) est parallèle à l'axe longitudinal.

3. Vis orthopédique selon la revendication 1, dans laquelle l'angle (α) entre la première face (112) et la crête (114) est au moins d'environ 90°.

4. Vis orthopédique selon la revendication 1, dans laquelle un rayon est constitué soit au moins entre la crête (114) et la première face (112), soit entre la crête (114) et la seconde face (116).

5. Vis orthopédique selon la revendication 1, dans laquelle la périphérie (106) de la tige (102) définit en outre une seconde forme dentée (120) espacée de la première forme dentée mentionnée (110) ; et dans laquelle la périphérie définit en outre une racine (122) placée entre la première forme dentée mentionnée et la seconde forme dentée.

6. Vis orthopédique selon la revendication 5, dans laquelle un rayon est constitué soit au moins entre la racine (122) et la première forme dentée mentionnée (110), soit entre la racine et la seconde forme dentée (120) .

7. Vis orthopédique selon la revendication 1, dans laquelle un élément arqué (136), de préférence sous la forme d'un rayon, relie au moins l'une des premières faces (112) à la crête (114) et la seconde face (116) à la crête (114).

8. Vis orthopédique selon la revendication 1, dans laquelle l'angle entre la première partie (144) de ladite première face (112) et ladite crête (114) est au moins d'environ 90°.

9. Vis orthopédique selon la revendication 1, dans laquelle une seconde partie (130) de la périphérie (106) de ladite tige (102) définit une surface lisse (132).

10. vis orthopédique selon la revendication 1, dans laquelle la périphérie (106) de ladite tige (102) est généralement cylindrique.

11. Vis orthopédique selon la revendication 1, qui comprend une tête (134) s'étendant de ladite tige (102) et opposée à la première extrémité mentionnée (104).

12. Vis orthopédique selon la revendication 1, dans laquelle la forme filetée (110) s'étend de façon hélicoïdale sur la périphérie (106) de ladite tige (102) sur au moins deux tours.

13. Ensemble de clou intramédullaire (200) à utiliser dans un canal médullaire d'un os long (2), ledit ensemble comprenant :
un clou (202) à placer au moins partiellement dans le canal médullaire, ledit clou comprenant une ouverture (204) dans celui-ci ; et
une vis (100) selon l'une quelconque des revendications 1 à 12.
